(19) Europäisches Patentamt
European Patent Office
Office européen des brevets

(11) EP 2 468 738 B1

(12) EUROPEAN PATENT SPECIFICATION

(45) Date of publication and mention of the grant of the patent:
**10.12.2014 Bulletin 2014/50**

(21) Application number: **10809843.5**

(22) Date of filing: **03.08.2010**

(51) Int Cl.:
***C07D 295/02*** *(2006.01)* ***C08K 5/529*** *(2006.01)*
***C08L 23/00*** *(2006.01)* ***C09K 21/12*** *(2006.01)*
***C08K 5/52*** *(2006.01)*

(86) International application number:
**PCT/JP2010/063072**

(87) International publication number:
**WO 2011/021498 (24.02.2011 Gazette 2011/08)**

# (54) (POLY) PIPERAZINE PYROPHOSPHATE POWDER AND MANUFACTURING METHOD THEREFOR

(POLY)PIPERAZINPYROPHOSPHATPULVER UND HERSTELLUNGSVERFAHREN DAFÜR

(POLY)PYROPHOSPHATE DE PIPÉRAZINE EN POUDRE ET SON PROCÉDÉ DE FABRICATION

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **20.08.2009 JP 2009191295**

(43) Date of publication of application:
**27.06.2012 Bulletin 2012/26**

(73) Proprietor: **SAKAI CHEMICAL INDUSTRY CO., LTD.**
**Sakai-ku**
**Sakai-shi**
**Osaka 590-8502 (JP)**

(72) Inventors:
- **OKITA Hiromasa**
  **Sakai-shi**
  **Osaka 590-0985 (JP)**
- **TSUJIMOTO Hideo**
  **Sakai-shi**
  **Osaka 590-0985 (JP)**
- **MURAKAMI Yasuyuki**
  **Sakai-shi**
  **Osaka 590-0985 (JP)**

(74) Representative: **Hart-Davis, Jason et al**
**Cabinet Beau de Loménie**
**158, rue de l'Université**
**75340 Paris Cedex 07 (FR)**

(56) References cited:
**WO-A1-2005/037806** **JP-A- 11 130 413**
**JP-A- 2003 221 389** **US-A- 3 810 850**
**US-A- 4 599 375**

- **JENSEN ET AL: "Piperazinium dihydrogen phosphate, C4H12N2(H2PO4)2: Synthesis, <31>P CP/MAS NMR, structural and thermal investigations", SOLID STATE SCIENCES, ELSEVIER, PARIS, FR, vol. 9, no. 1, 14 February 2007 (2007-02-14), pages 72-81, XP005889904, ISSN: 1293-2558, DOI: 10.1016/J.SOLIDSTATESCIENCES.2006.11.004**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 468 738 B1

## Description

TECHNICAL FIELD

The present invention relates to powdery (poly)piperazine pyrophosphate and the method of producing the same.

BACKGROUND OF THE INVENTION

(Poly)piperazine pyrophosphate has attracted attention as a flame retardancy-imparting agent for a synthetic resin in view of its excellent flame retardancy.

Many methods of producing (poly) piperazine pyrophosphate have been already reported. A known example as a simple method of producing piperazine pyrophosphate includes a reaction between piperazine hydrochloride and sodium pyrophosphate in an aqueous solution to produce piperazine pyrophosphate as poorly water-soluble precipitate (Patent Document 1). Patent Document 2 discloses a method including a treatment of sodium pyrophosphate with hydrochloric acid, followed by a reaction of resultant pyrophosphoric acid with piperazine in an aqueous solution to produce piperazine pyrophosphate as poorly water-soluble precipitate.

Unfortunately, the above methods have some problem on generation of byproducts such as sodium chloride or piperazine pyrophosphate sodium salt. Solubility of sodium chloride in water is relatively low, and scarcely varies depending on change in temperature. Thus, once sodium chloride is present in a reaction mixture, it is difficult to remove it from the mixture by rinsing, etc. Also, generation of byproduct piperazine pyrophosphate sodium salts cause decrease in yield of the target product. Thus, conventional method of (poly)piperazine pyrophosphate has room for improvement on yield and purity of products.

As an alternative for dissolving the problems in a conventional method as mentioned above, Patent Document 3 discloses a method of producing (poly) piperazine pyrophosphate by dehydration condensation of piperazine diphosphate.

[References]

[Patent Documents]

[Patent Document 1] JP 48-88791 A
[Patent Document 2] US Patent No. 4,599,375
[Patent Document 3] JP 2005-120021 A

SOLID STATE SCIENCES, ELSEVIER, vol. 9, pages 72-81, 2007, discloses piperazinium dihydrogen phosphate, C4H12N2(H2PO4)2: Synthesis, 31 P CP/MAS NMR, structural and thermal investigations.

WO 2005/037806 A1 discloses piperazine pyrophosphate having a high purity and a manufacture of piperazine pyrophosphate, which involves carrying out dehydration of piperazine diphosphate.

SUMMARY OF THE INVENTION

- Problem to be solved by the Invention

The method disclosed in Patent Document 3, however, remains a problem in that produced powdery (poly)piperazine pyrophosphate is generally stained gray or brown. Stain of additional agents affects color tone of final products that contains the agents. Thus, a resin composition or a molded article that contains such stained powdery (poly)piperazine pyrophosphate may be undesirably colored, and therefore, the powdery (poly)piperazine pyrophosphate has been used only in a limited field as an additional agent. Therefore, it has been desired to provide powdery (poly)piperazine pyrophosphate whose color is almost white, which would not affect the color tone of final products.

In view of the state of the art, an object of the present invention is to provide, powdery (poly)piperazine pyrophosphate that is not accompanied by difficult-to-remove byproducts and is not tinged with a color that would adversely affect the coloring of the molded article. Another object of the present invention is to provide a resin composition and a molded article which contain the powdery (poly)piperazine pyrophosphate. Still another object of the present invention is to provide a method of the powdery (poly)piperazine pyrophosphate in an efficient manner and with a high yield.

- Means for solving the problem

A first aspect of the present invention relates to powdery (poly) piperazine pyrophosphate, as claimed in claim 1. The powdery material has a whiteness of 80 to 100 as a W value, and a yellowness of 0 to 5 as a YI value.
A second aspect of the present invention relates to a flame retardancy-imparting agent for a resin, comprising the above powdery (poly)piperazine pyrophosphate.
A third aspect of the present invention relates to a molded article formed from the flame retardant resin composition.
A fourth aspect of the present invention relates to a flame retardant resin composition, comprising
100 parts by mass of a resin, and
20 to 80 parts by mass of the above powdery (poly)piperazine pyrophosphate. The resin is preferably a polyolefin resin.
A fifth aspect of the present invention relates to a method of producing powdery (poly)piperazine pyrophosphate as claimed in claim 1, comprising the step of dehydration condensation of piperazine diphosphate to produce (poly)piperazine pyrophosphate, the step being performed under an inert gas atmosphere.
The inert gas is preferably nitrogen gas. The piperazine diphosphate may preferably be a product of a reaction between phosphoric acid and piperazine.

- Effect of the Invention

According to a method of the present invention, highly-pure, white powdery (poly)piperazine pyrophosphate is obtainable with a high yield, without need for special manufacturing apparatus, and also without need for increasing the number of total steps compared with conventional methods. Powdery (poly)piperazine pyrophosphate of the present invention is excellent in flame retardancy, and therefore, it is preferably used as a flame retardancy-imparting agent to be mixed in a resin composition or a molded article. Further, the powdery (poly) piperazine pyrophosphate has a white color, and does not adversely affect the coloring of molded articles. Thus, the powdery (poly) piperazine pyrophosphate can be used in a wide field as an additional agent for a resin.

BRIEF DESCRIPTION OF THE DRAWINGS

[Fig. 1] A photograph of the powder that was obtained in Example 1.
[Fig. 2] A photograph of the powder that was obtained in Comparative Example 1.
[Fig. 3] A photograph of the molded article that was obtained in Application Example 1.
[Fig. 4] A photograph of the molded article that was obtained in Comparative Application Example 1.

MODE FOR CARRING OUT THE INVENTION

The present invention will be described below in detail.

<Powdery (poly)piperazine pyrophosphate>

The powdery (poly)piperazine pyrophosphate according to a first aspect of the present invention has a whiteness of 80 to 100 as a W value, and a yellowness of 0 to 5 as a YI value.
The "(poly)piperazine pyrophosphate" used herein means a piperazine pyrophosphate represented by the following formula (I):

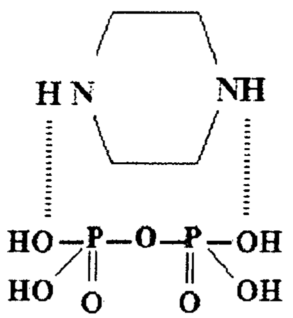

(I)

in the formula (I), dashed lines represents chemical bonds (for example, hydrogen bonds, covalent bonds, ion bonds, etc.), a poly(piperazine pyrophosphate) represented by the formula (II) :

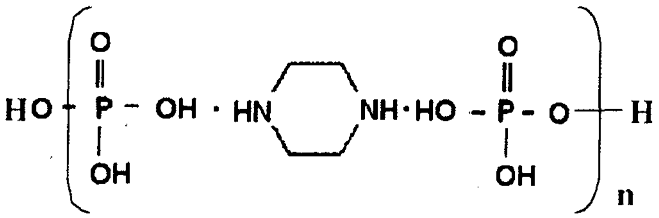

(I I)

in the formula, n is an integer of 2 to 100, and is a mixture of the piperazine pyrophosphate represented by the formula (I) and poly(piperazine pyrophosphate) represented by the formula (II).

Herein, the "W value" means a value of whiteness index, and the "YI" value means a value of yellowness index. Whiteness index (W) and yellowness index (YI) may be calculated by the equations below based on tristimulus values X, Y, and Z, where X represents a value of mainly sensing red among the three primary colors (red, blue, green), Y represents a value of sensing green, Z represents a value of sensing blue. Such whiteness index and yellowness index can be automatically measured by a color difference meter (for example, Type Z-300 A, manufactured by Nippon Denshoku Industries Co., Ltd.). Larger value of the whiteness index corresponds to higher whiteness. Larger value of the yellowness index corresponds to more increased yellowness.

Whiteness index (W) = Y + 800 ($x_n$-x) + 1700 ($y_n$-y) wherein x and y are chromaticity coordinates of the specimen in the XYZ colorimetric system, and $x_n$ and $y_n$ are chromaticity coordinates of the perfect reflecting diffuser in the XYZ colorimetric system (See JIS Z 8715 standard).

$$\text{Yellowness index (YI)} = 100(1.28X - 1.06Z)/Y$$

The powdery (poly)piperazine pyrophosphate of the present invention has a W value of within a range from 80 to 100. The W value is preferably within a range from 90 to 100, and more preferably within a range from 95 to 100.

The powdery (poly)piperazine pyrophosphate of the present invention has a YI value within a range from 0 to 5. The YI value is preferably within a range from 0 to 4, and more preferably within a range from 0 to 3.

The powdery (poly)piperazine pyrophosphate of the present invention has W and YI values of within the range as described above, and therefore it shows a white appearance when observed by the eye. Thus, molded articles that contain the powdery (poly)piperazine pyrophosphate can avoid problems or defects of products caused by staining.

(Method of producing powdery (poly)piperazine pyrophosphate)

Next, the method of producing powdery (poly)piperazine pyrophosphate of the present invention is described.

In the present invention, the (poly)piperazine pyrophosphate is obtainable by dehydration condensation of piperazine diphosphate.

Piperazine diphosphate may be produced through a reaction between two equivalents of orthophosphoric acid and one equivalent of piperazine as illustrated in the following reaction formula. The reaction can be conducted, although not limited to, in an aqueous solvent by heating at 70 to 90°C for 0.5 to 1 hour.

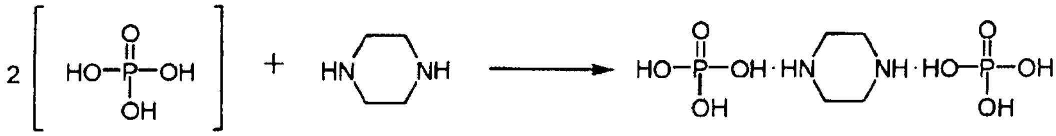

Then, dehydration condensation of the resultant piperazine diphosphate can be made to produce desired powdery (poly)piperazine pyrophosphate.

It is one of the characteristics of the present invention that the dehydration condensation step is performed under an inert gas atmosphere. If the dehydration condensation step is performed under an ordinary atmosphere, the resultant powdery (poly)piperazine pyrophosphate generally shows gray or blown color. On the contrary, when dehydration condensation is performed under an inert gas atmosphere according to the present invention, staining is prevented, and thus, white powdery (poly)piperazine pyrophosphate can be produced.

Examples of the inert gas include, but are not limited to, nitrogen, argon, helium, and carbon dioxide. Nitrogen is preferable since it is advantageous in high safety and low cost.

Although not limited to, dehydration condensation of piperazine diphosphate may be performed according to the following formula by, for example, heating piperazine diphosphate under nitrogen atmosphere at a temperature of between 230 to 320°C for 0.5 to 12 hours, or for 0.5 to 5 hours, or for 0.5 to 3 hours. Here, in the case where the dehydration condensation of piperazine diphosphate proceeds intermolecularly, poly(piperazine pyrophosphate) represented by the formula (II) is occurred, whereas in the case where the dehydration condensation of piperazine diphosphate proceeds intramolecularly, piperazine pyrophosphate represented by the formula (I) is occurred. The condition where intermolecular reaction is dominant or the condition where intramolecular reaction is dominant is appropriately selected based on general knowledge of a person skilled in the art. That is, the proportion of poly(piperazine pyrophosphate) and piperazine pyrophosphate in the reaction product may arbitrarily vary depending on reaction conditions. Typically, the reaction product is a mixture of poly(piperazine pyrophosphate) represented by the formula (II) and piperazine pyrophosphate represented by the formula (I).

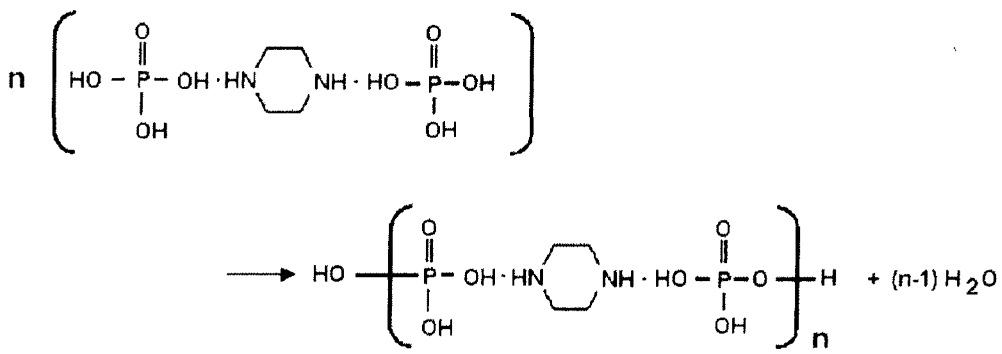

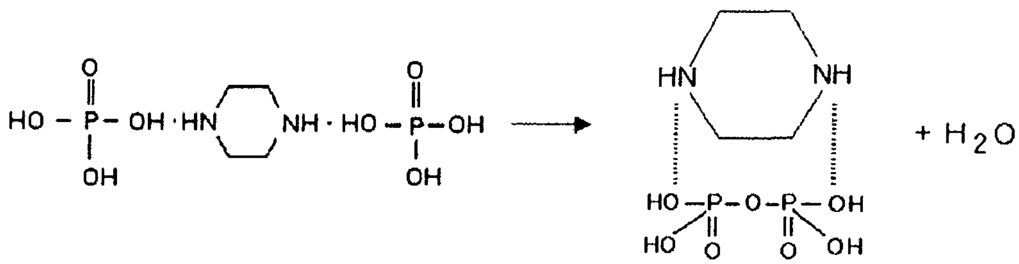

in the formula, dashed lines represent chemical bonds (for example, hydrogen bonds, covalent bonds, ion bonds, etc.).

Apparatus for dehydration condensation of piperazine diphosphate is not particularly limited as long as the apparatus endures heating and dehydration and allows the reaction under nitrogen atmosphere. For example, dehydration condensation may be performed using kneading apparatus equipped with a heater, hot-air drying apparatus, etc.

Specifically, the method using the kneading apparatus equipped with a heater includes the step of dehydration condensation of piperazine diphosphate at a heating temperature of 230 to 290 °C, a raw material-feeding rate of 20 to 100 kg/h, and a number of the rotation of 30 to 1,600 rpm. The kneading apparatus equipped with a heater is not particularly limited as long as it can produce large quantity of the target powdery (poly)piperazine pyrophosphate in an economically acceptable cost, and normal kneading apparatus may be used. Examples thereof include an extruder, a Henschel mixer, a Banbury mixer, a Plastomill, a KRC kneader, a vacuum kneader, and a pressurizing kneader. Among them, a Banbury mixer is suitable since it can promote the reaction efficiently.

Specifically, the method using the hot-air drying apparatus includes the step of dehydration condensation of piperazine diphosphate at a hot air temperature of 230 to 290°C. The hot-air drying apparatus is not particularly limited as long as it can produce large quantity of the target powdery (poly)piperazine pyrophosphate in an economically acceptable cost, and normal hot-air drying apparatus may be used. Examples thereof include a stirring dryer, a flash dryer, a circulation drier, a tray dryer, an atmosphere oven, a fluid bed dryer, a tube furnace, Drymeister(TM), an air dryer, Torusdisc(TM), Solidaire(TM), and a spray drier.

The powdery (poly)piperazine pyrophosphate produced by dehydration condensation of piperazine diphosphate contains little impurities, and is excellent in physical properties such as heat resistance and water resistance. Further, the powdery (poly) piperazine pyrophosphate of the present invention having W and YI values of within the range as

described above shows a white appearance when observed by the eye. Thus, molded articles that contain the powdery (poly)piperazine pyrophosphate can avoid problems or defects of products caused by staining.

The powdery (poly)piperazine pyrophosphate of the present invention is excellent in heat resistance and water resistance, and free of problematic staining. Thus, it is suitable to use as a flame retardancy-imparting agent for a resin. The flame retardancy-imparting agent for a resin which contains the powdery (poly)piperazine pyrophosphate constitutes one aspect of the present invention.

The flame retardancy-imparting agent of the present invention may be used in combination with various resins. Although not particularly limited, specific examples of the resin include thermoplastic resins including polyolefins or olefin copolymers, for example, α-olefin polymers such as polypropylene, high-density polyethylene, low-density polyethylene, linear low-density polyethylene, polybutene-1, and poly(3-methylpentene), or copolymers such as ethylene-vinyl acetate copolymer, ethylene-propylene copolymer; halogen-containing resins such as polyvinyl chloride, polyvinylidene chloride, chlorinated polyethylene, chlorinated polypropylene, polyvinylidene fluoride, chlorinated rubbers, vinyl chloride/vinyl acetate copolymer, vinyl chloride/ethylene copolymer, vinyl chloride/vinylidene chloride copolymer, vinyl chloride/vinylidene chloride/vinyl acetate terpolymer, vinyl chloride/acrylate copolymer, vinyl chloride/maleate copolymer, vinyl chloride/cyclohexyl maleimide copolymer; petroleum resins; coumarone resin; polystyrene; polyvinyl acetate; acrylic resins; copolymers of styrene and/or α-methyl styrene with other monomers (for example, maleic acid anhydride, phenylmaleimide, methyl methacrylate, butadiene, acrylonitrile, etc.), for example, AS resin, ABS resin, MBS resin, heat-resistant ABS resin, etc.; poly(methyl methacrylate); polyvinyl resins such as polyvinyl alcohol, poly(vinyl formal), and poly(vinyl butyral); linear polyesters such as polyethylene terephthalate and polybutylene terephthalate; polyphenyleneoxide; polyamides such as polycaprolactam and polyhexamethylene adipamide; polycarbonate; polycarbonate/ABS resin; branched polycarbonate; polyacetal; polyphenylene sulfide; polyurethane; cellulose resins; or blends of these thermoplastic resins; and thermosetting resins such as phenol resin, urea resin, melamine resin, epoxy resin, and unsaturated polyester resins. Among them, polyolefin resins are particularly preferable.

The flame retardant resin composition containing a resin and the powdery (poly)piperazine pyrophosphate which is a flame retardancy-imparting agent also constitutes one aspect of the present invention. The flame retardant resin composition of the present invention contains 20 to 80 parts by mass of the powdery (poly)piperazine pyrophosphate relative to 100 parts by mass of the resin. The lower limit of the amount of the powdery (poly)piperazine pyrophosphate in the composition is preferably 25 parts by mass, and more preferably 30 parts by mass, and the upper limit is preferably 60 parts by mass, and more preferably 40 parts by mass relative to 100 parts by mass of the resin.

The flame retardant resin composition of the present invention may contain, in addition to the powdery (poly)piperazine pyrophosphate, other flame retardants such as melamine pyrophosphate, poly(piperazine phosphate), poly(melamine phosphate), poly(phosphoric acid amide), or phosphates, phosphoric ester amides, and compounding ingredients such as polysiloxane compounds, metal oxides, silicon dioxide, or higher fatty carboxylic acids. Here, the amount of the other flame retardants to be mixed in the composition is preferably 0 to 100 parts by mass, and more preferably 10 to 100 mass relative to 100 parts by mass of the powdery (poly)piperazine pyrophosphate of the present invention. The amount of the compounding ingredients to be mixed in the composition is preferably 0 to 50 parts by mass, and more preferably 5 to 50 parts by mass relative to 100 parts by mass of the resin. The powdery (poly)piperazine pyrophosphate and the other flame retardants and/or the compounding ingredients may mixed together to form a flame retardant composition in advance of addition to the resin.

Such a flame retardant resin composition that contains one resin selected from the examples of resins listed above and the powdery (poly)piperazine pyrophosphate of the present invention is excellent in heat resistance, and has a white color. Thus, the resin composition is preferable in that color arrangement using pigments may be freely made, and the composition can be widely used. Such a flame retardant resin composition constitutes one aspect of the present invention.

The flame retardant resin composition of the present invention may be formed into a molded article by a normal molding method such as injection molding, extrusion molding, and inflation molding Such a molded article also constitutes one aspect of the present invention. The molded article of the present invention is not limited in its shape. Examples of the molded article include power plugs, connectors, sleeves, boxes, electric wire coatings, tape substrates, tubes, sheets, and films.

When injection-molded articles such as electric wire parts is produced as the molded article of the present invention, injection molding may be performed in a condition of a cylinder temperature of about 190°C, and a head temperature of about 190°C. The apparatus for injection molding may be a normal injection molder for molding PVC resin or the like.

## EXAMPLES

The present invention is described further in detail by way of the examples. These examples are for the purpose of illustration and should not limit the scope of the invention. In the following Examples and Comparative Examples, "%"

means % by mass unless otherwise noted.

<Measurement of whiteness and yellowness>

The whiteness (W value) and yellowness (YI value) of powdery (poly)piperazine pyrophosphate were measured using a simultaneous photometric spectrophotometer (manufactured by Nippon Denshoku Industries Co., Ltd., SQ-2000). For powdery samples, about 5 g of a sample was weighed in a cell, and tested. For sheet materials, 1.6-mm thick sheets for flame retardant test were used.

<Measurement of the temperature at which 5% mass loss occurred>

A temperature at which 5% mass loss occurred (A temperature at which mass loss of the sample reached 5%) was determined by measuring the TG (Thermogravimetry) of the powdery (poly)piperazine pyrophosphate in a thermo gravimetry/differential thermal analyzer (Seiko Instruments Inc., EXSTAR6300). A sample was weighed 7 $\pm$ 1 mg, and the TG was measured under air atmosphere at a temperature rise speed of 5°C/min.

<Oxygen index test (in accordance with the standard JIS K 7291) >

A test specimen having a length of 125 mm, a width of 6 mm, and a thickness of 3 mm was vertically supported, and ignited the upper terminal of the specimen by flame from a burner. After the upper terminal was burned like candle, the flame was removed, and then measurement of time of combustion and combusted length was soon started. Thus, minimum concentration of oxygen required for maintaining the burning for 3 minutes or 50 mm of length (together with nitrogen concentration at the time) was determined.

<Flame retardancy UL-94V test>

A test specimen having a length of 127 mm, a width of 12.7 mm, and a thickness of 1.6 mm was vertically supported. Flame from a burner was first contacted with the lower terminal of the specimen for 10 seconds. Then, the flame was removed, and the time until which the flame of the test specimen was extinguished was measured. Simultaneously with the extinction, the second flame contact was conducted for 10 seconds, and the time until which the flame was extinguished was measured similarly to the first measurement. Also, whether cotton placed below the test specimen was ignited by the cinder fallen from the test specimen was observed.

Tested samples were classified into flame classes according to UL-94 standard in view of the combustion time after the first and the second flame contacts, and presence or absence of ignition of cotton, etc. Flame class V-0 represents the highest flame retardancy, and the flame retardancy decreases in the order of V-0, V-1, and V-2. When a sample does not correspond to any classes among V-0 to V-2, the sample was ranked as "NR".

<Methods of producing powdery (poly)piperazine pyrophosphates>

[Example 1]

In a Banbury mixer (manufactured by MORIYAMA Co., Ltd., DSO.5-3GHH-E , volume: 2L), 200 g of piperazine diphosphate was mixed with heating for 1 to 3 hours under nitrogen atmosphere at a heating temperature of 240 to 270°C and a screw rotation of 30 to 100 rpm to produce 198 g (yield: 99%) of (poly) piperazine pyrophosphate as white powder. Here, nitrogen was aerated from the start of heating to the end of cooling (12 L/hour).

[Example 2]

In a tube type atmosphere oven (manufactured by Motoyama K. K. , Type: NLT-2035D), 3 g of piperazine diphosphate was mixed with heating for 1 to 3 hours under nitrogen atmosphere at a heating temperature of 240 to 270°C to produce 3 g (yield: 100%) of (poly)piperazine pyrophosphate as white powder. Here, nitrogen was aerated from the start of heating to the end of cooling (12 L/hour).

[Example 3]

In a Solidaire(TM) (manufactured by Hosokawa Micron, Type: Solidaire SJ), 20 kg of piperazine diphosphate was mixed with heating for 0.5 to 1 hour under nitrogen atmosphere at a heating temperature of 240 to 270°C to produce 18 kg (yield: 90%) of polypiperazine pyrophosphate as white powder. Here, nitrogen was aerated from the start of heating

to the end of cooling (20 L/hour).

[Comparative Example 1]

In a Banbury mixer (manufactured by MORIYAMA Co., Ltd., DS0.5-3GHH-E), 200 g of piperazine diphosphate was mixed with heating for 1 to 3 hours under air atmosphere at a heating temperature of 240 to 270°C and a screw rotation of 30 to 100 rpm to produce 190 g (yield: 95%) of (poly)piperazine pyrophosphate.

[Comparative Example 2]

In a tube type atmosphere oven (manufactured by Motoyama K. K. , Type: NLT-2035D), 3 g of piperazine diphosphate was mixed with heating for 1 to 3 hours under air atmosphere at a heating temperature of 240 to 270°C to produce 3 g (yield: 100%) of (poly)piperazine pyrophosphate.

Appearance, whiteness, yellowness, and the temperature at which 5% mass loss occurred of each of (poly)piperazine pyrophosphates obtained in Examples 1 to 3 and Comparative Examples 1 and 2 were determined according to the method as described above. Results are shown in Table 1.

[Table 1]

| | Example 1 | Example 2 | Example 3 | Comp. Ex. 1 | Comp. Ex. 2 |
|---|---|---|---|---|---|
| Powder: Appearance | White | White | White | Brown | Brown |
| Powder: Whiteness (W value) | 97.48 | 96.24 | 95.49 | 73.09 | 86.57 |
| Powder: Yellowness (YI value) | 1.85 | 1.85 | 1.89 | 15.17 | 9.08 |
| The temperature at which 5% mass loss occurred (°C) | 300 | 300 | 300 | 300 | 300 |
| *Comp. Ex. : Comparative Example | | | | | |

[Application Example and Comparative Application Example]

To 70 parts by mass of polypropylene resin (manufactured by Prime Polymer: a grade for injection molding), 2 parts by mass of calcium stearate (lubricant) and 30 parts by mass of powdery (poly)piperazine pyrophosphate were added, and then mixed, to prepare a polypropylene resin composition. The polypropylene resin composition was kneaded in a Labo Plastomill (TM) (manufactured by Toyo Seiki Seisaku-sho, Ltd., 10C100) at a temperature between 160 and 200°C. The resultant molded article was pelletized using a grinder (manufactured by DIAKO SEIKI Seisaku-sho., DAS-14). The pellet was injection-molded at 190°C to prepare a 1.6 mm-thick test specimen. The whiteness and yellowness of the obtained test specimen were measured. The results are shown in Table 2. The images of the powders and the molded articles are shown in Figs. 1 to 4. Flame retardancy of the test specimen was also tested.

[Table 2]

| | Application Example 1 | Comp. Application Example 1 |
|---|---|---|
| Polypropylene resin | 70 | 70 |
| (Poly)piperazine pyrophosphate (powder obtained in Example 1) | 30 | |
| (Poly)piperazine pyrophosphate (Powder obtained in Comparative Example 1) | | 30 |
| Calsium Stearate | 2 | 2 |
| Molded Article: Appearance | White | Gray |
| Molded Article: Whiteness (W value) | 89.33 | 67.87 |
| Molded Article: Yellowness (YI value) | 5.56 | 17.30 |
| UL-94 | V-0 | V-0 |

(continued)

| | Application Example 1 | Comp. Application Example 1 |
|---|---|---|
| Oxygen Index (L. O. I.) | 36.0 | 36.0 |
| * Coinp. Application Example: Comparative Application Example | | |

Further, results of whiteness, yellowness, the temperature at which 5% mass loss occurred, and appearance, which are listed in Tables 1 and 2 , and the images of Figs. 1 to 4 were summarized in Table 3.

[Table 3]

| | Example 1 Powder | Comparative Example 1 Powder |
|---|---|---|
| Photograph | Fig. 1 | Fig. 2 |
| Properties of Powder | Whiteness (W value) : 97.48<br>Yellowness (YI value): 1.85<br>Appearance: White<br>The temperature at which 5% mass loss occurred : 300 °C | Whiteness (W value) : 73.09<br>Yellowness (YI value): 15.17<br>Appearance: Brown<br>The temperature at which 5% mass loss occurred: 300 °C |
| | Application Example 1 Sheet | Comparative Application Example 1 Sheet |
| Photograph | Fig. 3 | Fig. 4 |
| Properties of Sheet | Whiteness (W value): 89.33<br>Yellowness (YI value): 5.56<br>Appearance: White | Whiteness (W value) : 67.87<br>Yellowness (YI value): 17.30<br>Appearance: Gray |

As is apparent from Tables 1 and 3, the powdery (poly)piperazine pyrophosphate produced by the method according to the present invention, which involves a reaction under inert atmosphere, showed higher whiteness and lower yellowness than conventional products, which was produced by a conventional method in which whole process was performed in the air.

As is also apparent from Tables 2 and 3, a molded article formed from the flame retardant resin composition that contained the powdery (poly)piperazine pyrophosphate of the present invention showed higher whiteness and apparently lower yellowness than the molded article that contained the powdery (poly)piperazine pyrophosphate produced by a conventional method.

As illustrated in Figs. 1 to 4, observation by the eye revealed that the powdery (poly) piperazine pyrophosphate of the present invention and the molded article that contained powdery (poly)piperazine pyrophosphate had a higher whiteness and a lower yellowness than conventional ones that had been produced by a method in which whole process was performed in the air.

As is apparent from Table 1, the powdery (poly) piperazine pyrophosphate of the present invention showed a temperature at which 5% mass loss occurred of 300°C, which was the same as the conventionally obtained powdery (poly)piperazine pyrophosphate. Since the temperature at which 5% mass loss occurred of piperazine diphosphate, which was a starting material, is 255°C, the result that the temperature at which 5% mass loss occurred of 300°C means that dehydration condensation was occurred in the production method of the present invention, and powdery (poly)piperazine pyrophosphate was synthesized.

As is apparent from Table 2, molded article made from the flame retardant resin composition that contained the powdery (poly) piperazine pyrophosphate of the present invention showed almost the same flame retardancy as the molded article that contained conventionally-produced powdery (poly)piperazine pyrophosphate.

Thus , dehydration condensation of piperazine diphosphate was performed under an inert gas atmosphere according to the method of the present invention enables to provide powdery (poly)piperazine pyrophosphate with high whiteness and low yellowness.

## Claims

1. Powdery (poly)piperazine pyrophosphate,

which has a whiteness of 80 to 100 as a W value, and a yellowness of 0 to 5 as a YI value, wherein said (poly)piperazine pyrophosphate is a mixture between piperazine pyrophosphate represented by formula (I)

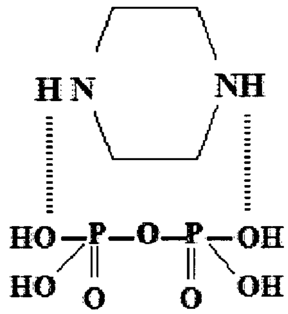

(I)

wherein in formula (I) dashed lines represents chemical bonds, and poly(piperazine pyrophosphate) represented by formula (II),

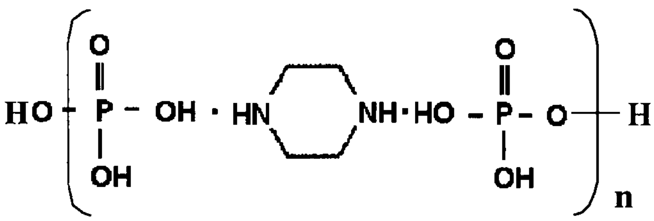

(II)

wherein in formula (II) n is an integer of 2 to 100.

**2.** A flame retardancy-imparting agent for a resin, comprising the powdery (poly)piperazine pyrophosphate according to claim 1.

**3.** A flame retardant resin composition, comprising
100 parts by mass of a resin, and
20 to 80 parts by mass of the powdery (poly)piperazine pyrophosphate according to claim 1.

**4.** The flame retardant resin composition according to claim 3,
wherein the resin is a polyolefin resin.

**5.** A molded article formed from the flame retardant resin composition according to claim 3 or 4.

**6.** A method of producing powdery (poly)piperazine pyrophosphate according to claim 1, comprising the step of dehydration condensation of piperazine diphosphate to produce (poly)piperazine pyrophosphate, the step being performed under an inert gas atmosphere,
wherein the dehydration condensation is performed using a kneading apparatus equipped with a heater at a heating temperature of 230 to 290 °C and a number of the rotation of 30 to 1,600 rpm.

**7.** The method according to claim 6,
wherein the inert gas is nitrogen gas.

**8.** The method according to claim 6 or 7,
wherein the piperazine diphosphate is a product of a reaction between orthophosphoric acid and piperazine.

## Patentansprüche

**1.** Puderiges (Poly)piperazinpyrophosphat,

das eine Weiße von 80 bis 100 als einen W-Wert und einen Gelbstich von 0 bis 5 als einen YI-Wert, aufweist, wobei das (Poly)piperazinpyrophosphat eine Mischung aus durch die Formel (I) dargestelltem Piperazinpyrophosphat,

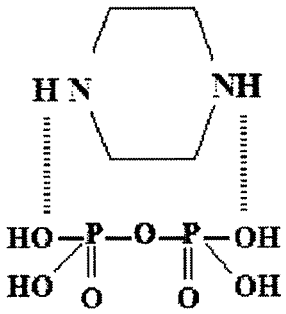

(I)

wobei in der Formel (I) die gestrichelten Linien chemische Bindungen darstellen,
und durch die Formel (II) dargestelltem (Poly)piperazinpyrophosphat ist

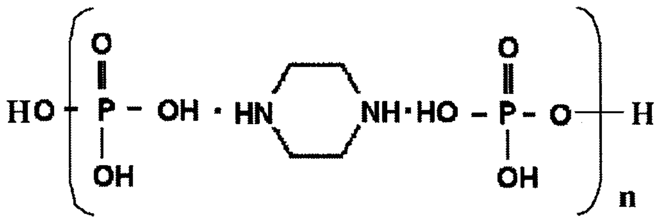

(II)

wobei in Formel (II) n eine ganze Zahl von 2 bis 100 ist.

**2.** Flammhemmungsgewährleistungsmittel für ein Harz, umfassend das pudrige (Poly)piperazinpyrophosphat nach Anspruch 1.

**3.** Flammhemmende Harzzusammensetzung, umfassend
100 Massenanteile eines Harzes und
20 bis 80 Massenanteile des pudrigen (Poly)piperazinpyrophosphates nach Anspruch 1.

**4.** Flammenhemmende Harzzusammensetzung nach Anspruch 3,
wobei das Harz ein Polyolefinharz ist.

**5.** Gussartikel, der aus der flammhemmenden Harzzusammensetzung nach Anspruch 3 oder 4 hergestellt ist.

**6.** Verfahren zum Herstellen eines pudrigen (Poly)piperazinpyrophosphates nach Anspruch 1, umfassend den Schritt der Dehydrationskondensation von Piperazindiphosphat, um (Poly)piperazinpyrophosphat herzustellen, wobei der Schritt unter einer inerten Gesamtatmosphere durchgeführt wird,
wobei die Dehydrationskondensation unter Verwendung einer Knetvorrichtung, die mit einer Heizvorrichtung ausgerüstet ist, bei einer Heiztemperatur von 230 bis 290 °C und einer Rotationsanzahl von 30 bis 1600 UpM durchgeführt wird.

**7.** Verfahren nach Anspruch 6,
wobei das inerte Gas Stickstoffgas ist.

**8.** Verfahren nach Anspruch 6 oder 7,
wobei das Piperazindiphosphat ein Produkt einer Reaktion zwischen Orthophosphorsäure und Piperazin ist.

**Revendications**

**1.** Pyrophosphate de (poly)pipérazine pulvérulent,
lequel présente un indice de blanc de 80 à 100 comme une valeur W, et un indice de jaune de 0 à 5 comme une valeur YI,
dans lequel ledit pyrophosphate de (poly)pipérazine est un mélange entre du pyrophosphate de pipérazine représenté par la formule (I)

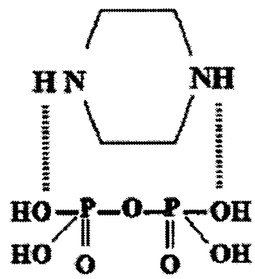

(I)

dans lequel dans la formule (I) les lignes en pointillés représentent des liaisons chimiques,
et du poly(pyrophosphate de pipérazine) représenté par la formule (II),

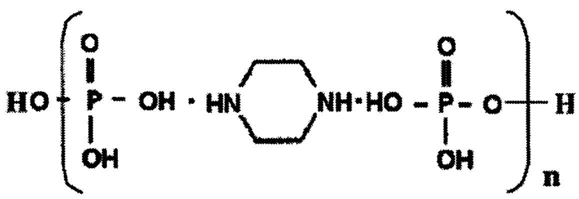

(II)

dans lequel dans la formule (II) n est un nombre entier de 2 à 100.

**2.** Agent communiquant un caractère ignifuge pour une résine, comprenant le pyrophosphate de (poly)pipérazine pulvérulent selon la revendication 1.

**3.** Composition de résine ignifuge, comprenant
100 parties en masse d'une résine, et
de 20 à 80 parties en masse du pyrophosphate de (poly)pipérazine pulvérulent selon la revendication 1.

**4.** Composition de résine ignifuge selon la revendication 3,
dans laquelle la résine est une résine de polyoléfine.

**5.** Article moulé formé à partir de la composition de résine ignifuge selon la revendication 3 ou 4.

**6.** Procédé de production de pyrophosphate de (poly)pipérazine pulvérulent selon la revendication 1, comprenant l'étape de déshydratation condensation de diphosphate de pipérazine pour produire du pyrophosphate de (poly)pipérazine, l'étape étant réalisée sous une atmosphère de gaz inerte,
dans lequel la déshydratation condensation est réalisée en utilisant un appareil de malaxage équipé d'un dispositif de chauffage à une température de chauffage de 230 à 290°C et un nombre de rotation de 30 à 1 600 tr/min.

**7.** Procédé selon la revendication 6,
dans lequel le gaz inerte est de l'azote gazeux.

**8.** Procédé selon la revendication 6 ou 7,
dans lequel le diphosphate de pipérazine est un produit d'une réaction entre l'acide orthophosphorique et la pipérazine.

Fig. 1

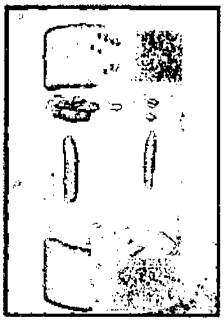

Fig. 2

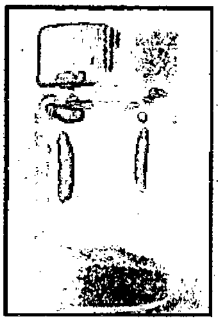

Fig. 3

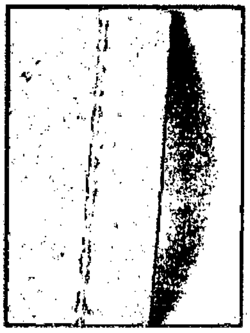

Fig. 4

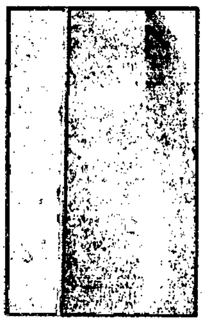

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 48088791 A
- US 4599375 A
- JP 2005120021 A
- WO 2005037806 A1

### Non-patent literature cited in the description

- SOLID STATE SCIENCES. ELSEVIER, 2007, vol. 9, 72-81